# NEUE EUROPÄISCHE PATENTSCHRIFT

(11) **EP 1 198 446 B2**
(45) Veröffentlichungstag und Bekanntmachung des Hinweises auf die Entscheidung über den Einspruch: **30.08.2006**
(45) Hinweis auf die Patenterteilung: 21.05.2003
(21) Anmeldenummer: 00952982.7
(22) Anmeldetag: 29.06.2000
(51) Int. Cl.: C07C 51/46, C07C 51/44, C07C 51/48, C07C 53/02, C07C 53/08

(54) **VERFAHREN ZUR TRENNUNG UND REINIGUNG EINES WÄSSRIGEN GEMISCHES AUS DEN HAUPTKOMPONENTEN ESSIGSÄURE UND AMEISENSÄURE**
METHOD FOR SEPARATING AND PURIFYING AN AQUEOUS MIXTURE THAT MAINLY CONSISTS OF ACETIC ACID AND FORMIC ACID
PROCEDE DE SEPARATION ET DE PURIFICATION D'UN MELANGE AQUEUX CONSTITUE PRINCIPALEMENT D'ACIDE ACETIQUE ET D'ACIDE FORMIQUE

(30) Priorität: 22.07.1999 DE 19934410
(43) Veröffentlichungstag der Anmeldung: 24.04.2002
(73) Patentinhaber: Consortium für elektrochemische Industrie GmbH, 81379 München (DE)
(72) Erfinder: RÜDINGER, Christoph, D-82319 Starnberg (DE); VOIT, Harald, Herbert, D-84571 Reischach (DE); HALLMANN, Michael, A-5122 Hochburg-Ach (AT); GÜNALTAY, Mehmet, D-84547 Emmerting (DE); REIL, Barbara, D-84547 Emmerting (DE); EBERLE, Hans-Jürgen, D-81477 München (DE)
(74) Vertreter: Rimböck, Karl-Heinz
(86) Internationale Anmeldenummer: PCT/EP2000/006092
(87) Internationale Veröffentlichungsnummer: WO 2001/007391

(56) Entgegenhaltungen:
- EP-A- 0 732 320
- GB-A- 735 867
- GB-A- 771 991
- GB-A- 771 992
- GB-A- 788 931
- GB-A- 1 062 555
- US-A- 2 861 923
- US-A- 3 555 083
- US-A- 5 399 751
- US-A- 5 662 780
- Chemistry and Industry, September 23, 1967, pp. 1590-92
- Environmental Progress, vol. 14, no. 1, 1995, pp. 61-64
- Derwent Abstract JP 07291890
- Derwent Abstract ZA 9202219
- Chem. Eng. Comm., 1995, vol. 136, pp. 161-176
- Chemical Engineering Progress, vol. 59, no. 10, 1963, pp. 65-68

## Beschreibung

Die Erfindung betrifft ein Verfahren zur Trennung und Reinigung eines wäßrigen Reaktionsgemisches aus den Hauptkomponenten Essigsäure und Ameisensäure.

Bei der Herstellung von Essigsäure durch katalytische Oxidationsreaktionen von gesättigten und/ oder ungesättigten Kohlenwasserstoffen wie beispielsweise der Gasphasenoxidation von C₄-Kohlenwasserstoffen, fallen Produktströme an, die als Hauptkomponenten Essigsäure, Ameisensäure und Wasser in unterschiedlichen Zusammensetzungen enthalten.

Zur weiteren Aufarbeitung müsseri diese Produktströme in ihre Einzelkomponenten getrennt werden. Eine destülative Trennung eines temären Säure-Wasser Gemisches aus Essigsäure, Ameisensäure und Wasser in seine Reinkomponenten bereitet beispielsweise erhebliche Probleme, da das System neben dem binären Wasser-Ameisensäure-Maximumazeotrop zusätzlich ein temäres Wasser-Ameisensäure-Essigsäure-Sattelazeotrop enthält.

Enthält ein derartiges Gemisch eine hohe Wasserkonzentration, so ergibt sich bei der destillativen Trennung ein enormer zusätzlicher Energiebedarf, da das gesamte Wasser als niedrigstsiedende Komponente über den Kopf einer Kolonne destilliert werden muß.

Hunsmann und Simmrock (Chemie-Ing. -Tech., 38,1966) empfehlen zurTrennungvon wäßrigen Gemischen mit einem Essigsäuregehalt von größer 60 Gew.-% und einem Ameisensäuregehalt von 5 Gew.-% die Anwendung der Azeotropdestillation zur Erleichterung der Trennung und zur Reduzierung der dabei benötigten Energie. Als Azeotropschleppmittel für die Entwässerung wird Ethyl-n-Butylether vorgeschlagen. Das Azeotrop aus Wasser und Schleppmittel siedet bei ca. 91 °C und enthält etwa 10 Gew.-% Wasser. Das Schleppmittel Ethyl-n-Butyletherbildet dabei kein Azeotrop mit Ameisensäure und Essigsäure.

E. Lloyd Jones beschreibt in Chemistry and Industry (1967), S. 1590 ff. die Vorteile der schon seit 1883 bekannten Lösungsmittelextraktion zur Trennung von Wasser und Essigsäure. Die Kombination einer Gegenstromextraktion mit einem mit Wasser sowohl eine Mischungslücke aufweisendem als auch ein leichtsiedendes Azeotrop bildendem Lösungsmittel und folgender Azeotroprektifikation mit diesem Lösungsmittel wird als deutlich vorteilhaft gegenüber der alleinigen Azeotroprektifikation zur Aufkonzentrierung von 50%-iger Essigsäure beschrieben. Die Gewinnung eines trockenen Säurestromes beim Vorliegen einer gemischten wässerigen Säurelösung wird hingegen als deutlich komplizierter aber theoretisch machbar beschrieben. Für die weitere Auftrennung eines solchen trockenen Stromes gemischter Säuren in die einzelnen Komponenten, insbesondere in Essigsäure und Ameisensäure werden jedoch keine konkreten und vorteilhaften Ausgestaltungen beschrieben.

In DE-A 1204214 wird zur Abtrennung von Ameisensäure die Azeotroprektifikation mit n-Butylchlorid als Schleppmittel empfohlen. Nachteilig an diesem Verfahren ist die Verwendung von chlorierten Kohlenwasserstoffen als Schleppmittel.

Aus US-A 5633402 ist ein Verfahren zurTrennung von binären Gemischen aus Ameisensäure und Essigsäure mittels Azeotropdestillation bekannt. Als Schleppmittel für die Ameisensäure wird dabei Methylformat verwendet. Eine Abtrennung von Wasser wird in diesem Verfahren nicht beschrieben.

Aus DE-A 4426132, EP-A 0635474, DE-A 19610356 (US-A 5662780), sind verschiedene Verfahren zur Reinigung und zur Entwässerung von Essigsäure mittels Azeotropen mit unterschiedlichen Schleppmitteln bekannt. Keines dieser Verfahren beschreibtjedoch die Entwässerung eines Gemisches aus Essigsäure und Ameisensäure.

Aus US-A 5173156, US-A 5006205, US-A 4877490 und US-A 4935100 sind Verfahren zur Entwässerung von Ameisensäure mittels Extraktivrektifikation bekannt. Dabei werden als Schleppmittel beispielsweise Cyclohexanon, Oxalsäure, Decansäure und Methylsalicylat genannt.

EP-A 156309 (CA-A 1238919) und EP-A 12321 (US-A 4262140) beschreiben die Entwässerung von Ameisensäure über Extraktivrektifikation mit Carboxamiden als Hilfsstoffe. Keines dieser Verfahren beschreibtjedoch die Entwässerung eines Gemisches aus Essigsäure und Ameisensäure.

Aus dem "Process Economics Program" Report No. 37A (1973) des Stanford Research Institute ist ein Verfahren zur Trennung eines wäßrigen Gemisches aus etwa 42 Gew.-% Essigsäure und 2 Gew.-% Ameisensäure bekannt. Das wäßrige Gemisch wird dabei durch Gegenstromextraktion mit Diisopropylether aufkonzentriert. In der Entwässerungs- und Lösungsmittelrückgewinnungskolonne wird das Wasser als Azeotrop aus Wasser und Diisopropylether über Kopf abdestilliert. Das Sumpfprodukt, ein Gemisch aus Essigsäure und Ameisensäure mit ca. 0,12 Gew.-% Wasser wird durch Azeotroprektifikation weiter aufgetrennt. Als Schleppmittel für die Ameisensäure wird.Benzol verwendet. Von großem Nachteil an diesem Verfahren ist die geringe Qualität der abgetrennten Ameisensäure, die noch ca. 1 Gew.-% Essigsäure, ca. 2 Gew.-% Wasser und ca. 7 Gew.-% Benzol enthält. Die Verwendung von Benzol in diesem Verfahren und der Restgehalt an Benzol in der Ameisensäure machen dieses Verfahren jedoch unattraktiv.

Alle im Stand der Technik bekannten Verfahren sind entweder nur dazu geeignet binäre Mischungen wie Essigsäure/Wasser, Ameisensäure/Wasser, und Essigsäure/Ameisensäure zufriedenstellend zu trennen, oder nur für wäßrige. Säuregemische wirtschaftlich anwendbar, in denen eine hohe Konzentration an Säure (> 60 Gew.-%) vorliegt. Weiterhin sind einige der bekannten Verfahren durch Ihren Einsatz von Benzol oder chlorierten Kohlenwasserstoffen nach heutigen Sicherheits- und Umweltgesichtspunkten nicht mehr akzeptabel.

Es bestand daher die Aufgabe ein Verfahren zur Trennung eines ternären, wäßrigen Gemisches aus Säuren in seine Reinkomponenten bereitzustellen, daß die im Stand der Technik genannten Nachteile nicht besitzt.

Es wurde nun gefunden, daß die Trennung und Reinigung eines Gemisches aus den Hauptkomponenten Essigsäure, Ameisensäure, Wasser und Hochsiedern (im weiteren Rohsäure genannt) besonders gut durchführbar ist, wenn in einem ersten Schritt mittels eines Lösungsmittels in einem Kreisverfahren extrahiert wird und anschließend der Extraktstrom, überwiegend bestehend aus Lösungsmittel, Essigsäure, Ameisensäure, Hochsiedern und Wasser, in einer Folge von Destillationsschritten in die Bestandteile Lösungsmittel, das zur Extraktion zurückgeführt wird, Wasser, Ameisensäure, Essigsäure und Hochsieder aufgetrennt wird, und der Raffinatstrom in einem weiteren Destillationsschritt mittels einer Lösungsmittelstripperkolonne vom Lösungsmittel befreit wird.

Gegenstand der Erfindung ist ein Verfahren zur Trennung und Reinigung eines wässrigen Gemisches aus den Hauptkomponenten Essigsäure, Ameisensäure und Schwersiedern durch Extraktion in einem Extraktor (7), mittels eines Lösungsmittels in einem Kreisverfahren, wobei der Raffinatstrom mit einem Großteil des Wassers einer Lösungsmittelstripperkolonne (11) zur Auskreisung des Wassers zugeführt wird und der Extraktstrom in eine Lösungsmitteldestillationskolonne (8) geleitet wird, dadurch gekennzeichnet, dass aus der Lösungsmitteldestillationskolonne (8) in einem ersten Schritt über Kopf eine Mischung (A), bestehend aus Wasser und Lösungsmittel, über den Sumpf eine Mischung (B) bestehend aus Essigsäure, Ameisensäure und Hochsiedern abgetrennt wird, wobei die Lösungsmitteldestillationskolonne (8) so betrieben wird, dass noch geringe Mengen an Wasser in Mischung (B) verbleiben, die Mischung (B) nach Abtrennung der Ameisensäure in Kolonne (29), die mit einem Seitenabzug ausgerüstet ist, aus dem ein Teilstrom enthaltend Wasser, Essigsäure und Ameisensäure abgezogen und zum Extraktor (7) zurückgeführt wird, anschließend in einer Essigsäuredestillationskolonne (5) in reine Essigsäure und Hochsieder aufgetrennt wird, und die Mischung (A) einem Phasentrenner (25) zugeführt wird, wobei die wässrige Phase mit Restanteilen an Lösungsmittel zur Lösungsmittelstripperkolonne (11), und die organische Phase zum Extraktor (7) zurückgeführt wird.

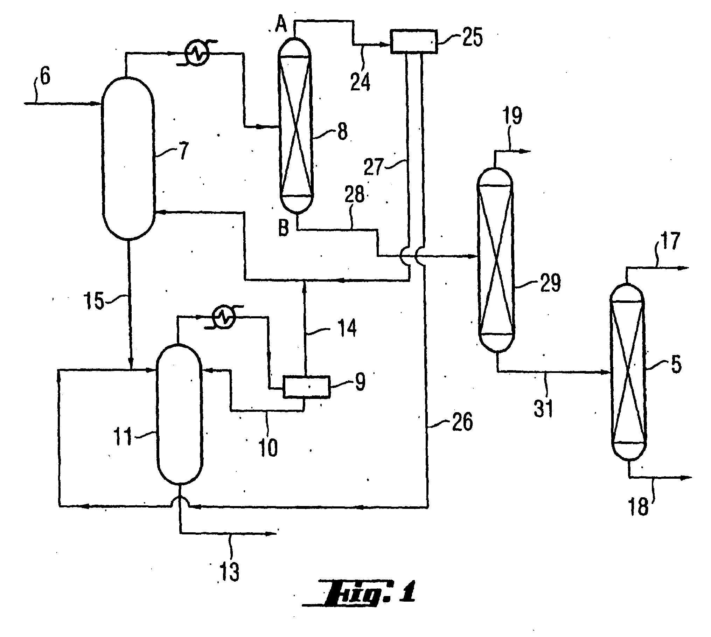

In der ersten Stufe (Extraktion) des Verfahrens (Fig. 1) wird die eingesetzte Rohsäure, bestehend aus wechselnden Anteilen von Essigsäure, Ameisensäure, Wasser und Schwersiedern einem Extraktor (7) über eine Leitung (6) zugeführt und mit einem Lösungsmittel kontaktiert. Der Extraktor (7) kann dabei einstufig oder bevorzugt mehrstufig aufgebaut sein. Der Lösungsmittelstrom kann in diesem Verfahren in Richtung des Stroms der Rohsäure gerichtet sein oder bevorzugt im Gegenstrom zur Rohsäure ausgelegt sein. Als Lösungsmittel können dabei Ether, Ester, Ketone, Alkohole, gesättigte, ungesättigte und cyclische Kohlenwasserstoffe mit 4 bis 8 Kohlenstoffatomen und deren Mischungen, bevorzugt Ether und Ester mit 4 bis 7 Kohlenstoffatomen, besonders bevorzugt Methyltertiärbutylether, Diisopropylether, Di-n-propylether, Ethylbutylether, Ethylacetat und Isopropylacetat, in einem Mischungsverhältnis zur Rohsäure (Volumen/Volumen) zwischen dem 0,5 bis 20-fachen, bevorzugt 1 bis 5-fachen, besonders bevorzugt 1,5 bis 3,5-fachen (Verhältnis Volumen/Volumen)verwendet werden. Die Extraktion kann in einem Temperatur- und Druckbereich stattfinden, in dem das Extraktionslösungsmittel und die Rohsäure in flüssiger Form und als getrennte Phasen, d.h. mit einer Mischungslücke, vorliegen. Bevorzugt ist ein Temperaturbereich von 0°C bis 60°C und ein Druckbereich von 1*10⁵ bis 20*10⁵ Pa.

Das aus dem Extraktor (7) erhaltene Raffinat wird über Leitung (15) der Lösungsmittelstripperkolonne (11) zugeführt, wo über den Sumpf reines Wasser entnommen wird (Leitung (13)). Das Kopfprodukt der Lösungsmittelstripperkolonne wird einem Phasentrenner (9) zugeführt. Die dort anfallende wäßrige Phase geht über Leitung (10) in den Kopf der Lösungsmittelstripperkolonne (11) zurück, die anfallende organische Phase wird über Leitung (14) dem Extraktor (7) zurückgeführt.

Das aus dem Extraktor (7) abgezogene Extrakt, enthaltend wechselnde Anteile an Lösungsmittel, Essigsäure, Ameisensäure, Wasser und Schwersieder wird vom Extraktor in eine Lösungsmitteldestillationskolonne (8) eingeleitet.

Die Lösungsmitteldestillationskolonne (8) kann unter Normaldruck, bevorzugt unter erhöhtem Druck betrieben werden.

Die Lösungsmitteldestillationskolonne (8) wird dabei vorzugsweise unter einem Druck von 1*10⁵ bis 50*10⁵ Pa, bevorzugt 1*10⁵ bis 25*10⁵, besonders bevorzugt 1*10⁵ bis 5*10⁵ Pa betrieben. In dieser Kolonne wird das Extrakt durch Destillation in zwei Teilströme aufgeteilt. Ein Teilstrom (Mischung (A)), bestehend aus einem Gemisch aus Lösungsmittel und Wasser wird dabei über Kopf der Kolonne entnommen und einem Phasentrenner (25) zugeführt (Leitung (24)). Die wäßrige Phase mit Restanteilen an Lösungsmittel wird über Leitung (26) abgetrennt, und der Lösungsmitteistripperkolonne (11), bevorzugt an der Einspeisestelle des Raffinats, zugeführt. Die organische Phase wird über Leitung (27) entnommen und dem Extraktor (7) zurückgeführt.

DerzweiteausKolonne(8)erhalteneTeilstrom (Mischung (B)), bestehend aus den Komponenten Essigsäure, Ameisensäure und Hochsiedem, wird aus der Lösungsmitteldestillationskolonne (8) über den Sumpf abgezogen und einer zwischengeschalteten Destillationskolonne (29) eingeleitet (Leitung (28)). Die Kolonne (29) wird ebenfalls unter normalem Druck, vorzugsweise unter erhöhtem Druck bei 1*10⁵ bis 50*10⁵ Pa, bevorzugt 1*10⁵ bis 25*10⁵ Pa, besonders bevorzugt 1*10⁵ bis 5*10⁵ Pa betrieben. Aus dieser Kolonne (29) wird über Kopf die reine Ameisensäure über Leitung (19) entnommen. Über den Sumpf wird ein ameisensäurefreies Gemisch aus Essigsäure und Hochsiedern entnommen und über Leitung (31) einer Essigsäuredestillationskolonne (5) zugeführt, in der die Auftrennung des Reststroms in reine Essigsäure und Schwersiedern erfolgt. Die Essigsäure wird am Kopf über Leitung (17) abgezogen und die Schwersieder werden am Kolonnensumpf über Leitung (18) abgetrennt.

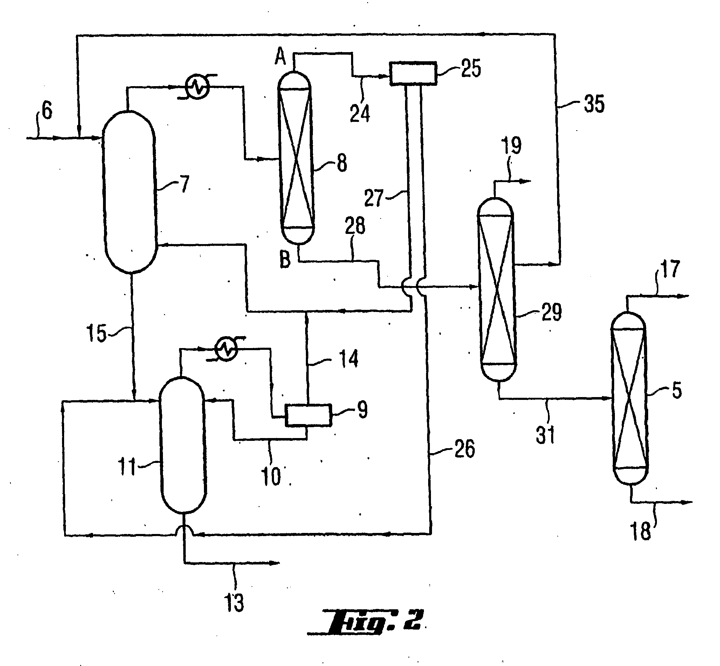

In einer besonderen Ausführungsform (Fig. 2) des erfindungsgemäßen Verfahrens wird die Lösungsmitteldestillationskolonne (8) so betrieben, daß aus dem Sumpf über Leitung (28) ein Teil des Wassers mitgeschleppt wird und zusammen mit der Essigsäure, der Ameisensäure und den Schwersiedern in die zwischengeschaltete Destillationskolonne (29) überführt wird. In diesem Fall wird aus der Destillationskolonne (29) über einen zusätzlichen Seitenabzug das Wasser mit geringen Anteilen an Essigsäure und Ameisensäure über Leitung (35) abgezogen und verworfen oder über Leitung (35) zum Rohsäureeingang (6) oder eine andere Stelle des Extraktors (7) zurückgeführt.

Bei dieser Ausführungsform wird die Trennaufgabe in der Lösungsmittelkolonne (8) gegenüber dem in Fig.1 aufgezeigten Verfahren durch das Mitschleppen von Wasser wesentlich vereinfacht. Weiterhin wird durch den zusätzlichen Seitenabzug an der Ameisensäuredestillationskolonne (29) auch die Trennung in reine Ameisensäure und das Sumpfprodukt, enthaltend Essigsäure und Schwersieder, vereinfacht.

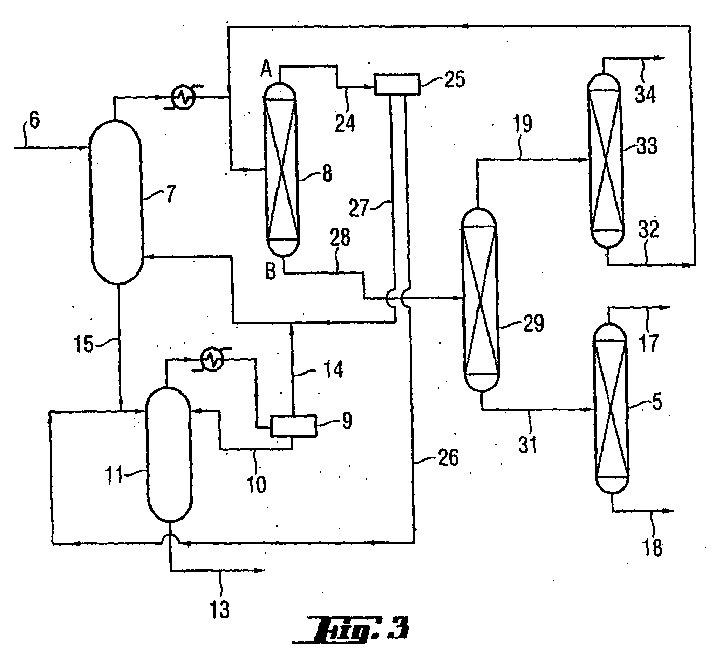

In einerweiteren Ausführungsform (Fig.3) des erfindungsgemäßen Verfahrens wird die Lösungsmitteldestillationskolonne (8) zur Erleichterung der Trennung ebenfalls so betrieben, daß der am Sumpf über Leitung (28) abgetrennte Teilstrom (Mischung (B)) noch geringe Mengen an Wasser enthält.
Dieses Sumpfprodukt, bestehend aus Essigsäure, Ameisensäure und Wasserresten wird in einer zwischengeschalteten Destillationskolonne (29) in ein ameisensäurefreies Sumfprodukt, enthaltend Essigsäure und Schwersieder und in ein gemischtes Kopfprodukt enthaltend Ameisensäure, Wasser und geringe Mengen Essigsäure aufgetrennt.

Das Kopfprodukt der Destillationskolonne (29), enthaltend Ameisensäure, Wasser und geringe Mengen an Essigsäure, wird anschließend über Leitung (19) der Ameisensäurereindestillationskolonne (33) zugeführt. Diese Kolonne (33) wird dabei mit einem niedrigerem Druck betrieben als die zwischengeschaltete Destillationskolonne (29). Die Druckdifferenz zwischen Kolonne (33) und Kolonne (29) beträgt dabei 0,1*10⁵ Pa bis 25*10⁵ Pa, bevorzugt 0.5*10⁵ Pa bis 5*10⁵ Pa. In der Ameisensäurereindestillationskolonne (33) wird der Produktstrom in reine Ameisensäure über Leitung (34) als Kopfprodukt und ein gemischtes Sumpfprodukt enthaltend, Essigsäure, Ameisensäure und Wasser aufgetrennt. Dieses Sumpfproduktwird über Leitung (32) zum Extraktstrom oder eine andere Einspeisestelle der Lösungsmitteldestillationskolonne (8) zurückgeführt.

Der Raffinatstrom (15) aus dem Extraktor (7) und diewäßrige Phase (26) aus dem Phasentrenngefäß (25) werden der Lösungsmittelstripperkolonne (11) zugeführt. Am Sumpf dieser Kolonne wird über Leitung (13) reines Wasser abgezogen. Das Kopfprodukt dieser Kolonne wird dem Phasentrenner (9) zugeführt. Die dort anfallende organische Phase wird dem Extraktors (7) zurückgeführt, die wäßrige Phase wird über Leitung (10) in den Kopf der Stripperkolonne (11) eingespeist.

Von großem Vorteil bei dieser Verfahrensausführung ist es, daß durch die zusätzliche Ameisensäurereindestillation (33) unter geringerem Druck als in der Trennkolonne (29) die Anforderungen an die Trennschärfe der Trennkolonne (29) deutlich geringer sind. Es ergibt sich dadurch eine deutliche Energieeinsparung bei stark verbesserter Ameisensäurereinheit gegenüber vergleichbaren Verfahren.
Weiterhin kann die Kondensationwärme der Destillationskolonne (29) im Sinne eines Wärmeverbunds zur Beheizung der Ameisendestillationskolonne (33) und der Lösungsmittelkolonne (11) verwendet werden. Die Reaktionswärme der diesen Trennverfahren vorgeschalteten Reaktionen, beispielsweise einer katalytischen Gasphasenoxidation von Kohlenwasserstoffen, kann ebenfalls zur Beheizung der Lösungsmitteldastillationskolonne (8), der Destillationskolonne (29), der Ameisendestillationskolonne (33) und der Essigsäuredestillatinoskolonne (5) verwendet werden.

In den folgenden Beispielen wird das erfindungsgemäße Verfahren unter Bezug auf die Abbildungen näher erläutert:

### Beispiel 1:

In einer Vorrichtung nach Ausführungsform Fig. 3 wurde dem Extraktor (7) (Gegenstromextraktionskolonne mit stationärer Edelstahlpackung, organische Phase dispergiert) über Leitung (6) ein Rohsäurestrom bestehend aus 12,9 kg/h Essigsäure, 2,6 kg/h Ameisensäure, 48,4 kg/h Wasser und 0,8 kg/h Hochsieder zugeführt. Über Leitung (27) und Leitung (14) wurde dem Extraktor (7) in stationären Zustand ein Lösungsmittelrückstrom enthaltend 135,4 kg/h Methyltertiärbutylether (MTBE), 4,0 kg/h Wasser, 0,5 kg/h Essigsäure und 0,2 kg/h Ameisensäurezugeführt. Der den Extraktor (7) verlassende Extraktstrom setzte sich aus 133,9 kg/h MT-BE, 13,1 kg/h Essigsäure, 8,1 kg/h Wasser, 2,6 kg/h Ameisensäure und 0,1 kg/h Hochsieder zusammen. Der den Extraktor (7) über Leitung (15) verlassende Raffinatstrom setzte sich aus 44,7 kg/h Wasser, 1,5 kg/h MTBE, 0,4 kg/h Essigsäure, 0,2 kg/h Ameisensäure und 0,7 kg/h Hochsieder zusammen.

Die Lösungsmitteldestillationskolonne (8) und die Destillationskolonne (29) wurden bei einem Druck von 2,75*10⁵ Pa betrieben. Die Ameisensäurereinkolonne (33) und Essigsäurereinkolonne (5) wurden bei einem Druck von 1*10⁵ Pa betrieben.

Am Sumpf der Lösungsmittelkolonne (8) wurde über Leitung (28) bei einer Temperatur von 147°C ein Strom, bestehend aus 13,4 kg/h Essigsäure, 3,7 kg/h Ameisensäure, 0,2 kg/h Wasser und 0,1 kg/h Hochsieder abgeführt. Aus dem Phasentrenner (25), der über Leitung (24) mit Kopf der Kolonne (8) verbunden war, wurde eine organische Phase über Leitung (27), enthaltend 133,8 kg/h MTBE, 0,5 kg/h Essigsäure, 0,2 kg/h Ameisensäure und 4,1 kg/h Wasser zum Lösungsmitteleingang des Extraktors (7) zurückgeführt. Der Abstrom der wäßriger Phase über Leitung (26) setzte sich aus 0,03 kg/h Essigsäure, 0,01 kg/h Ameisensäure, 4,1 kg/h Wasser und 0,1 kg/h MTBE zusammen.

Am Sumpf der Destillationskolonne (29) wurde bei einer Temperatur von 154,1 °C ein Strom enthaltend 12,6 kg/h Essigsäure und 0,1 kg/h Hochsieder über Leitung (31) entnommen. Am Sumpf der Essigsäurereinkolonne (5) wurde bei einer Temperatur von 143,6 °C über Leitung (18) ein Strom enthaltend 0,06 kg/h Essigsäure und 0,1 kg/h Hochsieder entnommen.

Der Abstrom aus dem Kopf der Ameisensäurereinkolonne (33) über Leitung (34) betrug 2,4 kg/h Ameisensäure. Über Leitung (32) wurden aus dem Sumpf der Ameisensäurereinkolonne (33) bei einer Temperatur von 106,2 °C ein Strom bestehend aus 0,8 kg/h Essigsäure, 1,3 kg/h Ameisensäure und 0,2 kg/h Wasser zum Eingang der Lösungsmitteldestillationskolonne (8) zurückgeführt.

Der Wasserabstrom über Leitung (1.3) aus dem Sumpf der Lösungsmittelstripperkolonne (11) enthielt 48,4 kg/h Wasser, 0,4 kg/h Essigsäure, 0,2 kg/h Ameisensäure und 0,7 kg/h Hochsieder. Der Rückstrom an organischer Phase über Leitung (14) aus dem Phasentrenngefäß (9) des Lösungsmittelstrippers (11) zum Lösungsmitteleingang des Extraktors (7) setzte sich aus 1,6 kg/h MTBE, 0,01 kg/h Essigsäure, 0,01 kg/h Ameisensäure und 0,05 kg/h Wasser zusammen.

Um die Rohsäuremischung in 2,4 kg/h an 99,9 Gew.-%iger Ameisensäure, 12,5 kg/h an 99,9 Gew-%iger Essigsäure und 49,6 kg/h an 97,5 Gew-%igem Wasser aufzutrennen wurde ohne Feedvorwärmung vor den Destillationskolonnen folgender Energieeinsatz benötigt:
Sumpfheizung Lösungsmitteldestillationskolonne (8): 20,5 kW
Sumpfheizung Trennkolonne (29): 10 kW
Sumpfheizung Ameisensäurereinkolonne (33): 5 kW
Sumpfheizung Essigsäurereinkolonne (5): 3,4 kW
Sumpfheizung Lösungsmittelstripperkolonne (11): 4 kW

Die Summe von 43 kW entspricht 2,87 kW pro kg Säure

### Beispiel 2:

In einer Vorrichtung nach Ausführungsform Fig. 3 wurde dem Extraktor (7) (Gegenstromextraktionskolonne mit stationärer Edelstahlpackung, organische Phase dispergiert) über Leitung (6) ein Rohsäurestrom enthaltend 12,9 kg/h Essigsäure, 2,6 kg/h Ameisensäure, 48,4 kg/h Wasser und 0,8 kg/h Hochsieder zugeführt. Über Leitung (27) und Leitung (14) wurde dem Extraktor (7) ein Lösungsmittelrückstrom enthaltend 135,4 kg/h Methyltertiärbutylether (MTBE), 4,0 kg/h Wasser, 0,5 kg/h Essigsäure und 0,2 kg/h Ameisensäure zugeführt. Der den Extraktor (7) verlassende Extraktstrom setzte sich aus 133,9 kg/h MTBE, 13,1 kg/h Essigsäure, 8,1 kg/h Wasser, 2,6 kg/h Ameisensäure und 0,1 kg/h Hochsiedern zusammen. Der den Extraktor (7) verlassende Raffinatstrom über Leitung (15) setzte sich aus 44,6 kg/h Wasser, 1,5 kg/h MTBE, 0,4 kg/h Essigsäure, 0,2 kg/h Ameisensäure und 0,7 kg/h Hochsiedern zusammen.

Die Lösungsmitteldestillationskolonne (8) und die Destillationskolonne (29) wurden bei einem Druck von 1,0*10⁵ Pa betrieben. Die Ameisensäurereinkolonne (33) wurde bei einem Druck von 0,25*10⁵ Pa betrieben. Die Essigsäurereinkolonne (5) wurden bei einem Druck von 1*10⁵ Pa betrieben.

Am Sumpf der Lösungsmittelkolonne (8) wurde bei einer Temperatur von 110°C, über Leitung (28) ein Strom, enthaltend 13,4 kg/h Essigsäure, 3,7 kg/h Ameisensäure, 0,2 kg/h Wasser und 0,1 kg/h Hochsieder abgeführt. Aus dem Phasentrenner (25), der über Leitung (24) mit dem Kopf'der Kolonne (8) verbunden war, wurde über Leitung (27) eine organische Phase, enthaltend 133,8 kg/h MTBE, 0,5 kg/h Essigsäure, 0,2 kg/h Ameisensäure und 4,0 kg/h Wasser zum Lösungsmitteleingang des Extraktors (7) zurückgeführt. Der Abstrom wäßriger Phase über Leitung (26) setzte sich aus 0,03 kg/h Essigsäure, 0,01 kg/h Ameisensäure, 4,0 kg/h Wasser und 0.1 kg/h MTBE zusammen.

Am Sumpf der Desliltationskolonne (29) wurde bei einer Temperatur von 117,8 °C über Leitung (31) ein Strom, bestehend aus 12,6 kg/h Essigsäure und 0,1 kg/h Hochsieder entnommen. Am Sumpf der Essigsäurereinkolonne (5) wurde über Leitung (18) bei einer Temperatur von 143,6 °C ein Strom, bestehend aus 0,1 kg/h Essigsäure und 0,1 kg/h Hochsieder entnommen.

Der Abstrom aus dem Kopf der Ameisensäurereinkolonne (33) über Leitung (34) betrug 2,4 kg/h Ameisensäure. Über Leitung (32) wurden aus dem Sumpf der Ameisensäurereinkolonne (33) bei einer Temperatur von 68,6 °C ein Strom, bestehend aus 0,8 kg/h Essigsäure, 1,3 kg/h Ameisensäure und 0,2 kg/h Wasser zum Eingang der Lösungsmitteldestillationskolonne (8) zurückgeführt.

Der Wasserabstrom über Leitung (13) aus dem Sumpf der Lösungsmittelstripperkolonne (11) enthielt 48,4 kg/h Wasser, 0,4 kg/h Essigsäure, 0,2 kg/h Ameisensäure und 0,7 kg/h Hochsieder. Der Rückstrom an organischer Phase über Leitung (14) aus dem Phasentrenngefäß (9) des Lösungsmittelstrippers (11) zum Lösungsmitteleingang des Extraktors (7) setzte sich aus 1,6 kg/h MTBE, 0,01 kg/h Essigsäure, 0,01 kg/h Ameisensäure und 0,01 kg/h Wasser zusammen.

Um die Rohsäuremischung in 2,4 kg/h an 99,9 Gew.-%iger Ameisensäure, 12,5 kg/h an 99,9 Gew-%iger Essigsäure und 49,6 kg/h an 97,5 Gew-%igem Wasser aufzutrennen wurde ohne Feedvorwärmung vor den Destillationskolonnen folgender Energieeinsatz benötigt:
Sumpfheizung Lösungsmitteldestillationskolonne (8): 30 kW
Sumpfheizung Trennkolonne (29): 18 kW
Sumpfheizung Ameisensäurereinkolonne (33): 3 kW
Sumpfheizung Essigsäurereinkolonne (5): 5 kW
Sumpfheizung Lösungsmittelstripperkolonne (11): 4,5 kW

Die Summe von 60,5 kW entspricht 4,05 kW pro kg Säure

### Beispiel 3 :

In einer Vorrichtung nach Ausführungsform Fig. 2 wurde dem Extraktor (7) (Gegenstromextraktionskolonne mit stationärer Edelstahlpackung, organische Phase dispergiert) über Leitung (6) ein Rohsäurestrom, bestehend aus 12,8 kg/h Essigsäure, 2,5 kg/h Ameisensäure, 48,6 kg/h Wasser und 0,8 kg/h Hochsieder zugeführt. Über Leitung (27) und Leitung (14) wurde dem Extraktor (7) ein Lösungsmittelrückstrom, bestehend aus 179,7 kg/h Methyltertiärbutylether (MTBE), 1,9 kg/h Wasser, 0,4 kg/h Essigsäure und 0,1 kg/h Ameisensäure zugeführt. Der den Extraktor (7) verlassende Extraktstrom setzte sich aus 178,3 kg/h MTBE, 13,1 kg/h Essigsäure, 9,8 kg/h Wasser, 2,6 kg/h Ameisensäure und 0,2 kg/h Hochsieder zusammen. Der den Extraktor (7) verlassende Raffinatstrom über Leitung (15) setzte sich aus 40,7 kg/h Wasser, 1,3 kg/h MTBE, 0,2 kg/h Essigsäure, 0,1 kg/h Ameisensäure und 0,6 kg/h Hochsieder zusammen.

Die Lösungsmitteldestillationskolonne (8) und die Destillationskolonne (29) wurden bei einem Druck von 2,75*10⁵ Pa betrieben. Die Essigsäurereinkolonne (5) wurde bei einem Druck von 1*10⁵ Pa betrieben.

Am Sumpf der Lösungsmittelkolonne (8) wurde über Leitung (28) bei einer Temperatur von 148,7°C ein Strom, enthaltend 12,6 kg/h Essigsäure, 2,4 kg/h Ameisensäure, 0,1 kg/h Wasser und 0,2 kg/h Hochsieder abgeführt. Der über Kopf durch Leitung (24) abgetrennte Teilstrom wurde dem Phasentrenner (25) zugeführt und dort aufgetrennt. Die erhaltene organische Phase, bestehend aus 177,9 kg/h MTBE, 0,4 kg/h Essigsäure, 0,1 kg/h Ameisensäure und 1,8 kg/h Wasser wurde über Leitung (27) in den Extraktor (7) zurückgeführt. Der Abstrom an wäßriger Phase über Leitung (26) setzte sich aus 0,03 kg/h Essigsäure, 0,02 kg/h Ameisensäure, 7,8 kg/h Wasser und 0,4 kg/h MTBE zusammen.

Am Sumpf der Destillationskolonne (29) wurde über Leitung (31) bei einer Temperatur von 154,2 °C ein Strom, bestehend aus 12,5 kg/h Essigsäure, 0,01 kg/h Ameisensäure und 0,1 kg/h Hochsieder entnommen. Am Sumpf der Essigsäurereinkolonne (5) wurde über Leitung (18) bei einer Temperatur von 150 °C ein Strom aus 0,04 kg/h Essigsäure und 0,1 kg/h Hochsiedern entnommen.

Der Abstrom aus dem Kopf der Destillationskollonne (29) über Leitung (19) enthielt 0,01 kg/h Essigsäure, 2,4 kg/h Ameisensäure und 0,01 kg/h Wasser. Über Leitung (35) wurden aus der Kolonne (29) ein Seitenabzugsstrom, enthaltend 0,05 kg/h Essigsäure, 0,8 kg/h Ameisensäure und 0,1 kg/h Wasser entnommen.

Der Wasserabstrom über Leitung (13) aus dem Sumpf der Lösungsmittelstripperkolonne (11) enthielt 48,5 kg/h Wasser, 0,3 kg/h Essigsäure, 0,08 kg/h Ameisensäure und 0,6 kg/h Hochsieder. Der Rückstrom an organischer Phase zum Extraktor über Leitung (14) aus dem Phasentrenngefäß (9) des Lösungsmittelstrippers (11) setzte sich aus 1,7 kg/h MTBE, 0,01 kg/h Essigsäure, 0,01 kg/h Ameisensäure und 0,05 kg/h Wasser zusammen.

Um die Rohsäuremischung in 1,6 kg/h an 98,6 Gew.-%iger Ameisensäure, 12,5 kg/h an 99,99 Gew.-%iger Essigsäure und 49,4 kg/h an 98,1 Gew.-%igem Wasseraufzutrennenwurdeohne Feedvorwärmungvor den Destillationskolonnen folgender Energieeinsatz benötigt:
Sumpfheizung Lösungsmitteldestillationskolonne (8): 22,5 kW
Sumpfheizung Trennkolonne (29): 10 kW
Sumpfheizung Essigsäurereinkolonne (5): 4 kW
Sumpfheizung Lösungsmittelstripperkolonne (11): 4,5 kW

Die Summe von 41 kW entspricht 2,9 kW pro kg Säure

## Patentansprüche

1. Verfahren zur Trennung und Reinigung eines wässrigen Gemisches aus den Hauptkomponenten Essigsäure, Ameisensäure und Schwersiedern durch Extraktion in einem Extraktor (7), mittels eines Lösungsmittels in einem Kreisverfahren, wobei der Raffinatstrom mit einem Großteil des Wassers einer Lösungsmittelstripperkolonne (11) zur Auskreisung des Wassers zugeführt wird und der Extraktstrom in eine Lösungsmitteldestillationskolonne (8) geleitet wird, **dadurch gekennzeichnet, dass** aus der Lösungsmitteldestillationskolonne (8) in einem ersten Schritt über Kopf eine Mischung (A), bestehend aus Wasser und Lösungsmittel, über den Sumpf eine Mischung (B) bestehend aus Essigsäure, Ameisensäure und Hochsiedern abgetrennt wird, wobei die Lösungsmitteldestillationskolonne (8) so betrieben wird, dass noch geringe Mengen an Wasser in Mischung (B) verbleiben, die Mischung (B) nach Abtrennung der Ameisensäure in Kolonne (29), die mit einem Seitenabzug ausgerüstet ist, aus dem ein Teilstrom enthaltend Wasser, Essigsäure und Ameisensäure abgezogen und zum Extraktor (7) zurückgeführt wird, anschließend in einer Essigsäuredestillationskolonne (5) in reine Essigsäure und Hochsieder aufgetrennt wird, und die Mischung (A) einem Phasentrenner (25) zugeführt wird, wobei die wässrige Phase mit Restanteilen an Lösungsmittel zur Lösungsmittelstripperkolonne (11), und die organische Phase zum Extraktor (7) zurückgeführt wird.

2. Verfahren zur Trennung und Reinigung eines wässrigen Gemisches aus den Hauptkomponenten Essigsäure, Ameisensäure und Schwersiedern durch Extraktion in einem Extraktor (7), mittels eines Lösungsmittels in einem Kreisverfahren, wobei der Raffinatstrom mit einem Großteil des Wassers einer Lösungsmittelstripperkolonne (11) zur Auskreisung des Wassers zugeführt wird und der Extraktstrom in eine Lösungsmitteldestillationskolonne (8) geleitet wird, **dadurch gekennzeichnet, dass** aus der Lösungsmitteldestillationskolonne (8) in einem ersten Schritt über Kopf eine Mischung (A), bestehend aus Wasser und Lösungsmittel, über den Sumpf eine Mischung (B) bestehend aus Essigsäure, Ameisensäure und Hochsiedern abgetrennt wird, wobei die Lösungsmitteldestillationskolonne (8) so betrieben wird, dass noch geringe Mengen an Wasser in Mischung (B) verbleiben, die Mischung (B) in einer zwischengeschalteten Destillationskolonne (29) in ein ameisensäurefreies Sumfprodukt, enthaltend Essigsäure und Schwersieder und in ein gemischtes Kopfprodukt enthaltend Ameisensäure, Wasser und geringe Mengen Essigsäure aufgetrennt wird, wobei das Sumpfprodukt aus Kolonne (29) in einer nachgeschalteten Essigsäuredestillationskolonne (5) in reine Essigsäure und Schwersieder aufgetrennt wird und das Kopfprodukt aus Kolonne (29) einer Ameisensäurereindestillationskolonne (33) zugeführt wird, wo es in reine Ameisensäure als Kopfprodukt und ein gemischtes Sumpfprodukt enthaltend, Essigsäure, Ameisensäure und Wasser aufgetrennt wird, welches zur Lösungsmitteldestillationskolonne (8) zurückgeführt wird und die Mischung (A) einem Phasentrenner (25) zugeführt wird, wobei die wässrige Phase mit Restanteilen an Lösungsmittel zur Lösungsmittelstripperkolonne (11), und die organische Phase zum Extraktor (7) zurückgeführt wird.

3. Verfahren nach Anspruch 2, **dadurch gekennzeichnet, dass** die Ameisensäurereindestillationskolonne (33) bei einem um 0,1*10⁵ bis 25*10⁵ Pa niedrigeren Druck als die zwischengeschaltete Destillationskolonne (29) betrieben wird.

4. Verfahren nach Anspruch 1 bis 3, **dadurch gekennzeichnet, dass** die Kondensationswärme der Destillationskolonne (29) zur Beheizung der Ameisensäuredestillationskolonne (33) und/oder der Lösungsmitteldestillationskolonne (11) verwendet wird.

5. Verfahren nach Anspruch 1 bis 4, **dadurch gekennzeichnet, dass** die Reaktionswärme der vorgeschalteten Reaktion zur Herstellung von Essigsäure durch katalytische Gasphasenoxidation von Kohlenwasserstoffen zur Beheizung einer oder mehrerer der Kolonnen umfassend, die Lösungsmitteldestillationskolonne (8), die Destillationskolonne (29), die Essigsäuredestillationskolonne (5) und die Ameisensäuredestillationskolonne (33), verwendet wird.

6. Verfahren nach Anspruch 1 bis 5, **dadurch gekennzeichnet, dass** die Lösungsmitteldestillationskolonne (8) unter Normaldruck betrieben wird.

7. Verfahren nach Anspruch 1 bis 6, **dadurch gekennzeichnet, dass** die Lösungsmitteldestillationskolonne (8) unter erhöhtem Druck zwischen 1*10⁵ und 50*10⁵ Pa betrieben wird.

8. Verfahren nach Anspruch 1 bis 7, **dadurch gekennzeichnet, dass** der Extraktor ein- oder mehrstufig betrieben wird.

9. Verfahren nach Anspruch 1 bis 8, **dadurch gekennzeichnet, dass** der Lösungsmittelkreislauf im Extraktor im Gegenstrom zur Rohsäure verläuft.

10. Verfahren nach Anspruch 1 bis 9, **dadurch gekennzeichnet, dass** als Lösungsmittel gesättigte, ungesättigte und/oder cyclische Kohlenwasserstoffe mit 4 bis 8 Kohlenstoffatomen eingesetzt werden.

11. Verfahren nach Anspruch 1 bis 10, **dadurch gekennzeichnet, dass** als Lösungsmittel eine oder mehrere Verbindungen aus der Gruppe umfassend Ether, Ester, Ketone, Kohlenwasserstoffe und Alkohole eingesetzt werden.

12. Verfahren nach Anspruch 1 bis 11, **dadurch gekennzeichnet, dass** als Lösungsmittel eine oder mehrere Verbindungen aus der Gruppe umfassend Methyltertiärbutylether, Diisopropylether, Di-n-propylether, Ethylbutylether, Ethylacetat und Isopropylacetat verwendet werden.

13. Verfahren nach Anspruch 1 bis 12, **dadurch gekennzeichnet, dass** die Extraktion bei Temperaturen von 0 bis 60°C und Drücken von 1*10⁵ bis 20*10⁵ Pa durchgeführt wird.

14. Verfahren nach Anspruch 1 bis 13, **dadurch gekennzeichnet, dass** das Lösungsmittel in einem Mischungsverhältnis zur Rohsäure (Volumen/Volumen) zwischen dem 0,5 und 20-fachen liegt.

15. Verfahren nach Anspruch 1 bis 14, **dadurch gekennzeichnet, dass** die zwischengeschaltete Destillationskolonne (29) bei einem Druck von 1*10⁵ Pa bis 50*10⁵ Pa betrieben wird.

## Claims

1. Process for the separation and purification of an aqueous mixture comprising the main components acetic acid, formic acid and high boilers by extraction with a solvent in an extractor (7) in a circulation process, in which the raffinate stream containing a major part of the water is fed to a solvent stripping column (11) for removal of the water and the extract stream is conveyed to a solvent distillation column (8), **characterized in that**, in a first step, a mixture (A) comprising water and solvent is separated off via the top of the solvent distillation column (8) and a mixture (B) comprising acetic acid, formic acid and high boilers is separated off via the bottom, with the solvent distillation column (8) being operated in such a way that small amounts of water remain in mixture (B), the formic acid is separated off from the mixture (B) in column (29) which is equipped with a side offtake from which a substream comprising water, acetic acid and formic acid is taken off and recirculated to the extractor (7) and the remaining mixture (B) is subsequently fractionated into pure acetic acid and high boilers in an acetic acid distillation column, and the mixture (A) is conveyed to a phase separator (25) from which the aqueous phase containing residual solvent is recirculated to the solvent stripping column (11) and the organic phase is recirculated to the extractor (7).

2. Process for the separation and purification of an aqueous mixture comprising the main components acetic acid, formic acid and high boilers by extraction with a solvent in an extractor (7) in a circulation process, in which the raffinate stream containing a major part of the water is fed to a solvent stripping column (11) for removal of the water and the extract stream is conveyed to a solvent distillation column (8), **characterized in that**, in a first step, a mixture (A) comprising water and solvent is separated off via the top of the solvent distillation column (8) and a mixture (B) comprising acetic acid, formic acid and high boilers is separated off via the bottom, with the solvent distillation column (8) being operated in such a way that small amounts of water remain in mixture (B), the mixture (B) is fractionated into a bottom product which is free of formic acid and comprises acetic acid and high boilers and a mixed top product comprising formic acid, water and small amounts of acetic acid in an intermediate distillation column (29), with the bottom product from column (29) being fractionated into pure acetic acid and high boilers in a downstream acetic acid distillation column (5) and the top product from column (29) being fed to a pure formic acid distillation column (33) where it is fractionated into pure formic acid as top product and a mixed bottom product which comprises acetic acid, formic acid and water and is recirculated to the solvent distillation column (A), and the mixture (A) is conveyed to a phase separator (25) from which the aqueous phase containing residual solvent is recirculated to the solvent stripping column (11) and the organic phase is recirculated to the extractor (7).

3. Process according to Claim 2, **characterized in that** the pure formic acid distillation column (33) is operated at a pressure which is from 0.1*10⁵ to 25*10⁵ Pa lower than that in the intermediate distillation column (29).

4. Process according to any of Claims 1 to 3, **characterized in that** the heat of condensation in the distillation column (29) is used for heating the formic acid distillation column (33) and/or the solvent distillation column (11).

5. Process according to any of Claims 1 to 4, **characterized in that** the heat of reaction of the upstream reaction for preparing acetic acid by catalytic gas-phase oxidation of hydrocarbons is used for heating one or more of the solvent distillation column (8), the distillation column (29), the acetic acid distillation column (5) and the formic acid distillation column (33).

6. Process according to any of Claims 1 to 5, **characterized in that** the solvent distillation column (8) is operated under atmospheric pressure.

7. Process according to any of Claims 1 to 6, **characterized in that** the solvent distillation column (8) is operated under a superatmospheric pressure of from 1*10⁵ to 50*10⁵ Pa.

8. Process according to any of Claims 1 to 7, **characterized in that** the extractor is operated in one or more stages.

9. Process according to any of Claims 1 to 8, **characterized in that** the solvent circuit in the extractor runs countercurrent to the crude acid.

10. Process according to any of Claims 1 to 9, **characterized in that** the solvent used comprises saturated, unsaturated and/or cyclic hydrocarbons having from 4 to 8 carbon atoms.

11. Process according to any of Claims 1 to 10, **characterized in that** the solvent used is one or more compounds selected from the group consisting of ethers, esters, ketones, hydrocarbons and alcohols.

12. Process according to any of Claims 1 to 11, **characterized in that** the solvent used is one or more compounds selected from the group consisting of methyl tert-butyl ether, diisopropyl ether, di-n-propyl ether, ethyl butyl ether, ethyl acetate and isopropyl acetate.

13. Process according to any of Claims 1 to 12, **characterized in that** the extraction is carried out at temperatures of from 0 to 60°C and pressures of from 1*10⁵ to 20*10⁵ Pa.

14. Process according to any of Claims 1 to 15, **characterized in that** the mixing ratio of solvent to crude acid (volume/volume) is from 0.5 to 20.

15. Process according to any of Claims 1 to 14, **characterized in that** the intermediate distillation column (29) is operated at a pressure of from 1*10⁵ Pa to 50*10⁵ Pa.

## Revendications

1. Procédé de séparation et de purification d'un mélange aqueux constitué principalement d'acide acétique, d'acide formique et de substances bouillant difficilement, par extraction dans un extracteur (7) au moyen d'un solvant dans un procédé en circuit fermé, le courant de raffinat contenant une grande partie de l'eau étant amené à une colonne d'élimination de solvant (11) pour faire sortir l'eau du circuit, et le courant d'extrait étant envoyé dans une colonne de distillation de solvant (8), **caractérisé en ce que**, dans une première étape, depuis la colonne de distillation de solvant (8), sont séparés en tête, un mélange (A) constitué d'eau et de solvant, et par le fond, un mélange (B) constitué d'acide acétique, d'acide formique et de substances de point d'ébullition élevé, la colonne de distillation de solvant (8) fonctionnant de sorte que de faibles quantités d'eau subsistent encore dans le mélange (B), le mélange (B) après séparation de l'acide formique dans la colonne (29), qui est équipée d'une évacuation latérale à partir de laquelle un courant partiel comprenant de l'eau, de l'acide acétique et de l'acide formique est soutiré et renvoyé à l'extracteur (7), est ensuite séparé dans une colonne de distillation d'acide acétique (5) en acide acétique pur et substances de point d'ébullition élevé, et le mélange (A) est amené à un séparateur de phases (25), la phase aqueuse comprenant des fractions résiduelles de solvant étant renvoyée vers la colonne d'élimination de solvant (11), et la phase organique vers l'extracteur (7).

2. Procédé de séparation et de purification d'un mélange aqueux constitué principalement d'acide acétique, d'acide formique et de substances bouillant difficilement, par extraction dans un extracteur (7) au moyen d'un solvant dans un procédé en circuit fermé, le courant de raffinat contenant une grande partie de l'eau étant amené à une colonne d'élimination de solvant (11) pour faire sortir l'eau du circuit, et le courant d'extrait étant envoyé dans une colonne de distillation de solvant (8), **caractérisé en ce que**, dans une première étape, de la colonne de distillation de solvant (8), sont séparés en têtes, un mélange (A) constitué d'eau et de solvant, et par le fond, un mélange (B) constitué d'acide acétique, d'acide formique et de substances de point d'ébullition élevé, la colonne de distillation de solvant (8) fonctionnant de sorte que de faibles quantités d'eau subsistent encore dans le mélange (B), le mélange (B) constitué des composants acide acétique, acide formique, substances bouillant difficilement et résidus d'eau est séparé dans une colonne de distillation intercalée (29) en un produit de fond exempt d'acide formique, contenant de l'acide acétique et des substances bouillant difficilement et en un produit de tête mélangé, contenant de l'acide formique, de l'eau et de faibles quantités d'acide acétique, le produit de fond de la colonne (29) étant séparé dans une colonne de distillation d'acide acétique (5) raccordée en aval, en acide acétique pur et en substances bouillant difficilement et le produit de tête de la colonne (29) étant amené à une colonne de distillation d'acide formique (33) où il est séparé en acide formique pur comme produit de tête et en un produit de fond mélangé contenant de l'acide acétique, de l'acide formique et de l'eau, produit de fond qui est renvoyé au courant d'extrait vers la colonne de distillation de solvant (8), et le mélange (A) est amené à un séparateur de phases (25), la phase aqueuse comprenant des fractions résiduelles de solvant étant renvoyée vers la colonne d'élimination de solvant (11), et la phase organique vers l'extracteur (7).

3. Procédé selon la revendication 2, **caractérisé en ce que** la colonne de distillation d'acide formique (33) fonctionne à une pression plus basse de 0,1*10⁵ à 25*10⁵ Pa que celle de la colonne de distillation intercalée (29).

4. Procédé selon les revendications 1 à 3, **caractérisé en ce que** la chaleur de condensation de la colonne de distillation (29) est utilisée pour le chauffage de la colonne de distillation d'acide formique (33) et/ou de la colonne de distillation de solvant (11).

5. Procédé selon les revendications 1 à 4, **caractérisé en ce que** la chaleur réactionnelle de la réaction se produisant en amont et destinée à produire de l'acide acétique par oxydation catalytique en phase gazeuse d'hydrocarbures, est utilisée pour le chauffage d'une ou plusieurs colonnes comprenant la colonne de distillation de solvant (8), la colonne de distillation (29), la colonne de distillation d'acide acétique (5) et la colonne de distillation d'acide formique (33).

6. Procédé selon les revendications 1 à 5, **caractérisé en ce que** la colonne de distillation de solvant (8) fonctionne sous la pression normale.

7. Procédé selon les revendications 1 à 6, **caractérisé en ce que** la colonne de distillation de solvant (8) fonctionne sous une pression augmentée entre 1*10⁵ et 50*10⁵ Pa.

8. Procédé selon les revendications 1 à 7, **caractérisé en ce que** l'extracteur fonctionne en extracteur mono- ou multi-étagé.

9. Procédé selon les revendications 1 à 8, **caractérisé en ce que** la circulation du solvant dans l'extracteur à lieu à contre-courant de l'acide brut.

10. Procédé selon les revendications 1 à 9, **caractérisé en ce que** des hydrocarbures saturés, insaturés et/ou cycliques, présentant 4 à 8 atomes de carbone sont utilisés comme solvant.

11. Procédé selon les revendications 1 à 10, **caractérisé en ce qu'**un ou plusieurs composés parmi le groupe comprenant les éthers, les esters, les cétones, les hydrocarbures et les alcools est (sont) utilisé(s) comme solvant.

12. Procédé selon les revendications 1 à 11, **caractérisé en ce qu'**un ou plusieurs composés parmi le groupe comprenant l'éther de méthyle et de butyle tertiaire, l'éther diisopropylique, l'éther di-n-propylique, l'éther d'éthyle et de butyle, l'acétate d'éthyle et l'acétate d'isopropyle est (sont) utilisé(s) comme solvant.

13. Procédé selon les revendications 1 à 12, **caractérisé en ce que** l'extraction est effectuée à des températures de 0 à 60°C et à des pressions de 1*10⁵ à 20*10⁵ Pa.

14. Procédé selon les revendications 1 à 15, **caractérisé en ce que** le solvant se trouve dans un rapport de mélange par rapport à l'acide brut (volume/volume) entre la moitié et le vingtuple.

15. Procédé selon les revendications 1 à 14, **caractérisé en ce que** la colonne de distillation intercalée (29) fonctionne à une pression de 1*10⁵ Pa à 50*10⁵ Pa.
